# EUROPÄISCHE PATENTSCHRIFT

(11) **EP 1 475 431 B1**
(45) Veröffentlichungstag und Bekanntmachung des Hinweises auf die Patenterteilung: **04.03.2009**
(21) Anmeldenummer: 04010064.6
(22) Anmeldetag: 28.04.2004
(51) Int. Cl.: C12N 1/16, C12N 1/26

(54) **Verfahren zur Produktion von Lipase**
Method for the production of lipase
Procédé de production de la lipase

(30) Priorität: 07.05.2003 DE 10320653
(43) Veröffentlichungstag der Anmeldung: 10.11.2004
(73) Patentinhaber: ARTECHNO, 5030 Gembloux (BE)
(72) Erfinder: Thonart, Philippe, 5081 Saint-Denis (BE); Destain, Jacqueline, 1450 Chastre (BE); Moreau, Benoit, 7022 Hyon (BE); Fickers, Patrick, 4200 Liège (BE); Weekers, Frédéric, 4122 Plainevaux (BE); Molitor, Jean-Pierre, 77760 Buthiers (FR); Weiss, Albrecht, Dr., 40764 Hilden (DE)
(74) Vertreter: Lerho, Marc J. A.

(56) Entgegenhaltungen:
- SHIMADA, YUJI ET AL.: "Induction of Geotrichum candidum lipase by long-chain fatty acids" JOURNAL OF FERMENTATION AND BIOENGINEERING, Bd. 74, Nr. 2, - 1992 Seiten 77-80, XP009032773

## Beschreibung

### Gebiet der Erfindung

Die Erfindung befindet sich auf dem Gebiet der mikrobiellen Produktion von Enzymen, speziell der Lipase und insbesondere der Lipase aus Hefen und betrifft ein Verfahren zur Produktion von extrazellulären Lipasen aus Mikroorganismen, insbesondere aus Hefen.
Des Weiteren betrifft die Erfindung die Verwendung von Fettsäureestern als Induktoren zur Produktion von Lipase aus Mikroorganismen.

### Stand der Technik

In der chemischen und biochemischen Synthese werden vermehrt Enzyme als Katalysatoren eingesetzt. So werden in vielen Fällen aufgrund der oft milderen Reaktionsbedingungen bereits in großtechnischen Verfahren Hydrolasen, speziell Lipasen (EC 3.1.1.3) zur Fettspaltung eingesetzt.
Diese Enzyme werden von unterschiedlichen Mikroorganismen produziert. Zur Isolierung der Enzyme folgt nach der Fermentation der Mikroorganismen ein aufwendiges Reinigungsverfahren.
Der Effektivität dieser Katalysatoren stehen oftmals die hohen Kosten der Produktion und der Isolierung gegenüber, so dass Forschungsgruppen immer wieder bestrebt sind die Ausbeuten an Enzymen zu erhöhen oder die Kosten der Produktion durch kostengünstige Nährmedien bei der Fermentation oder vereinfachte Isolierung so gering wie möglich zu halten. Eine Möglichkeit die Ausbeute an Enzymen zu erhöhen besteht darin, aus dem natürlichen Mikroorganismus durch Mutagenese Mutanten zu züchten bei denen eine erhöhte Produktion beobachtet werden kann. Zur Mutagenese eignen sich sowohl biologische als auch chemische Verfahren. Der Nachteil bei dieser Art der Ausbeuteerhöhung liegt in der aufwendigen und langwierigen Suche nach einer geeigneten Mutante.
Eine weitere, heute oftmals verwendete Methode besteht darin, die Mikroorganismen genetisch zu verändern und gezielt bestimmte Promotoren der codierenden Gene zu induzieren. Ein Nachteil bei diesem Verfahren ist die mangelnde Akzeptanz genetisch veränderter Prozesse und die Ablehnung von Produkten aus genetisch veränderten Prozessen. Oftmals beschrieben und aus der Literatur bekannt ist die Möglichkeit Tenside, Fette und Öle wie Olivenöl, Sojaöl, Acylglyceride wie beispielsweise beschrieben in EP 385 401 dem Fermentationsmedium beizumischen um die Produktion an Lipasen zu steigern. Bei diesen Verfahren ist die Ausbeutesteigerung oftmals zu gering.

Die Aufgabe der vorliegenden Erfindung hat nun darin bestanden, eine kostengünstige Variante zu finden um die Ausbeute an Lipase bei natürlichen Mikroorganismen oder bei deren Mutanten zu erhöhen.
SHIMADA, Yuji et al. befasste sich in "Induction of Geotrichum candidum Lipase by Long-Chain Fatty Acids", Journal of Fermentation and Bioengineering, Bd. 74, Nr. 2, 77-80 (1992) mit der Wirkung von Fettsäurekettenlängen auf extrazelluläre Lipaseproduktion aus dem Stamm *G. candidum.* Langkettige Fettsäuren und deren Ester (sowie Methylpalmitat und mit Ethanol verstreutes Methylstearat) bewirken eine Lipaseproduktion während mittel- und kurzkettige Fettsäuren und deren Ester dies nicht tun. Die Lipaseproduktion nimmt mit der Kettenlänge der Fettsäure zu, und langkettige ungesättigte Fettsäureester waren die wirksamsten (aber weniger al 80 U/ml).

### Beschreibung der Erfindung

Gegenstand der Erfindung ist ein Verfahren zur Produktion von extrazellulärer Lipase, durch Kultivierung von Mikroorganismen beim dem der Gehalt von Lipase in Kulturbeständen durch Zugabe von Fettsäureestern kurzkettiger einwertiger Alkohole als Induktoren erhöht wird.

Überraschenderweise wurde gefunden, dass die Zugabe von Fettsäureestern kurzkettiger einwertiger Alkohole als Induktoren die Produktionsrate an extrazellulärer Lipase bei der Kultivierung von Mikroorganismen erhöht.
Bei den zu kultivierenden Mikroorganismen des erfindungsgemäßen Verfahrens handelt es sich um die Hefe *Yarrowia lipolytica.* Als Induktor zur Erhöhung der Produktionsrate der erfindungsgemäßen Lipase wurden nun überraschenderweise Fettsäureester kurzkettiger einwertiger Alkohole gefunden, die ausgewählt sind aus der Gruppe die gebildet wird von Ester der Olsäure: Methyloleat und Ethyloleat. Der sogenannte Induktor wird in Mengen zwischen 0,5 und 5% v/v eingesetzt, bezogen auf das Fermentationsmedium.
In einer weiteren besonderen Ausführungsform der Erfindung handelt es sich bei den zu kultivierenden Hefen entweder um den Wildtyp oder um Mutanten. Ein bevorzugter Wildtyp ist der Stamm *Yarrowia lipolytica* CBS 6303. Die Herstellung der Mutanten kann nach chemischen oder biologischen Methoden erfolgen und wird bevorzugt durch N-methyl-N'-nitrosoguanidin vorgenommen wie es von Roblain et al. 1989 in Belg. J. Food Chem. 44; 79-82 beschrieben wurde. Zur Produktion der erfindungsgemäßen Lipase findet bevorzugt die Mutante *Yarrowia lipolytica* LGX 6481 Anwendung.

Im Sinne der Erfindung handelt es sich bei extrazellulärer Lipase um fettspaltende Enzyme die von Mikroorganismen produziert werden und aus dem Zellinneren in das Fermentationsmedium geschleust werden. Die Lipase wird nach bekannten Methoden isoliert und gereinigt. Bevorzugt folgt nach der Fermentation eine Zentrifugation des Nährmediums, sodass sich die extrazelluläre Lipase im Überstand befindet. Die erfindungsgemäße, vom Wildtyp und von Mutanten der Hefe *Yarrowia lipolytica* produzierte Lipase ist bevorzugt glycosyliert und hat ein Molekulargewicht zwischen 35.000 und 40.000 Dalton, insbesondere zwischen 37.000 und 38.000 Dalton und bevorzugt 37.664 Dalton. Die bevorzugte Lipase kann in mindestens drei unterschiedlichen isomeren Formen vorliegen deren isoelektrische Punkte bei 5,0; 5,2 und 5,4 liegen. Die bevorzugte Lipase hydrolysiert eine Vielzahl an Triglyceriden und Fettsäureestern und hydrolysiert insbesondere Ölsäureester. Im Sinne der Erfindung versteht man unter Kultivierung von Mikroorganismen die Fermentation in Schüttelkolben und/oder Fermentern unterschiedlicher Größe wobei Fermenter im 2 I, 20 I und 500 I Maßtstab bevorzugt sind. Der Fermentation ist eine Vorkultur in Schüttelkolben von beispielsweise 250 ml mit bevorzugt 50 ml Kulturmedium vorgeschaltet. Diese Vorkulturen werden dann zur Animpfung des Fermentationsmediums verwendet. Das Fermentationsmedium der Vorkultur besteht vevorzugt aus Glucose, Hefeextrakt und Pepton in veränderlichen Gewichtsanteilen und 5 % v/v Inoculum. Die Vorkultur wird bevorzugt 16 h bei 250 rpm und bei 29°C inkubiert. Das Fermentationsmedium des Fermenters besteht bevorzugt aus Malzextrakt, CaCO₃, MgSO₄, K₂HPO₄, Urea, Sojabohnenmehl und Olivenöl in veränderten Gewichtsanteilen mit 5 % v/v Inoculum. Es können im Sinne der Erfindung jedoch auch andere Bestandteile für die Fermentation eingesetzt werden, insbesondere solche, die speziell für die Kultivierung von Hefen Anwendung finden und bevorzugt kostengünstig sind. Die Fermentationsbedingungen sind den jeweiligen Fermentern angepasst und variieren je nach Größe und Hersteller der Fermenter. Bevorzugt wird die Hauptkultur 27 h bei 29°C und einem ph-Wert von 7 kultiviert. Beschrieben werden die Fermentationsbedingungen auch in:Destain et al, 1997, Biotechnology Letters 19, 2, 105-107.

Im Sinne der Erfingung vesteht man unter Induktoren solche Verbindungen, die nach Zugabe zur Fermenation die Produktion eines bestimmten Enzymes oder Metaboliten erhöhen. Die Induktoren werden bevorzugt je nach Bedarf des Mikroorganismus im fed-batch Verfahren zugegeben damit eine zu hohe Konzentration des Induktors zu Beginn der Fermentation ausgeschlossen werden kann, da diese toxische Auswirkungen auf den zu kultivierenden Mikroorganismus haben können. Der bevorzugte Induktor ist insbesondere Methyloleat beispielsweise Agrimul® ME 2231F der Firma Cognis GmbH & Co KG.Die erhöhte Produktionsrate wurde bestimmt durch Messung der Aktivität der Lipase durch Spaltung von Olivenöl einer bestimmten Emulsion bei pH7 und 37°C. Die molekulare Aktivität wird angegeben als Umsatz des Substrates in micromol pro Enzymmenge (mircromol) und Zeit (min). Eine Enzymeinheit U/ml (enzyme unitis) ist definiert als Enzymmenge, die pro Minute ein mircomol Substrat umsetzt. In einer bevorzugten Ausführungsform des erfindungsgemäßen Verfahrens werden die Induktoren zwischen 0,5 und 2,5 % v/v bezogen auf das Fermentationsmedium eingesetzt, wobei ein Gehalt von 0,5 % vv oder 1,0 % vv, oder insbesondere 2,5 % v/v bevorzugt ist. Die Induktoren können allein oder in Gemischen von den zwei oben genannten Estern eingesetzt werden wobei die jeweiligen Mengenanteile variieren können, Durch den Einsatz der Induktoren kann die Aktivität der Lipase von 1000 U/ml bis zu 2500-3000 U/ml in der Fermentationslösung gesteigert werden. Da es sich bei den erfindungsgemäßen Induktoren um Fettsäureester handelt, ist eine bevorzugte Ausführungsform der Erfindung ein Verfahren, bei dem der verwendete Induktor gleichzeitig als Kohlenstoffquelle bei der Kultivierung dient und damit an die Stelle der im Fermentationsmedium enthaltenen Fettsäuren, Fette oder Öle wie beispielsweise Ölsäure oder Gemische mit überwiegendem Anteil an Ölsäure tritt und diese ersetzt. In diesem Fall werden die Fettsäureester als Kohlenstoffquelle in Mengen zwischen 2,5 % v/v bis 5 % v/v eingesetzt. Im Sinne der Erfindung versteht man unter Fettsäureester kurzkettiger einwertiger Alkohole solche Ester, deren Alkohole eine C-Kettenlänge von 1 bis maximal 4 haben, wobei die Alkohole nur einwertig sind, die C-Ketten jedoch auch verzweigt sein können und die Alkohole damit nicht nur primär, sondern auch sekundär oder tertiär sein können. Es handelt sich um Methyl- oder Ethylester. Das fed-batch Verfahren wird insbesondere angewendet bei Ethyloleat als Induktor, da das bei der Hydrolyse entstehende Ethanol für die Hefe toxisch wirkt. Durch das fed-batch Verfahren wird jeweils nur soviel Ethyloleat in das Fernentationsmedium gegeben, dass die Produktion des Enzyms angeregt wird, die Menge an Ethanol jedoch noch nicht toxisch auf den Mikroorganismus wirkt.

Ein weiterer Gegenstand der Erfindung ist die Verwendung von Fettsäureestern kurzkettiger einwertiger Alkohole als Induktoren zur Produktion von extrazellulärer Lipase aus Mikroorganismen. Bei den verwendeten Fettsaureestern handelt es sich um Ester ausgewählt aus der Gruppe , die gebildet wird von Ester der Ölsäure, speziell Methyloleat und Ethyloleat, und insbesondere um Methyloleat, beispielsweise Agrimul®ME 2231F der Firma Cognis GmbH & Co KG. In einer bevorzugten Ausführungsform der erfindungsgemäßen Verwendung handelt es sich bei dem Mikroorganismus um die Hefe *Yarrowia lipolytica.* In einer weiteren besonderen Ausführungsform der Erfindung handelt es sich bei den zu kultivierenden Hefen entweder um den Wildtyp oder um Mutanten.

## Patentansprüche

1. Verfahren zur Produktion von extrazellulärer Lipase durch Kultivierung von einem Mikroorganismus, **dadurch gekennzeichnet, dass** :
- der Mikroorganismus aus der Hefe *Yarrowia lipolytica* besteht ;
- ein Fettsäureester kurzkettiger einwertiger Alkohole ausgewählt aus der Gruppe, die von Methyloleat und Ethyloleat gebildet wird, als Induktor zugegeben wird, so dass der Gehalt an extrazellulärer Lipase in Kulturüberständen erhöht wird, und
- der sogennante Induktor in Mengen zwischen 0,5 und 5% v/v eingesetzt wird, bezogen auf das Fermentationsmedium.

2. Verfahren nach Anspruch 1, **dadurch gekennzeichnet, dass** es sich bei der Hefe *Yarrowia lipolytica* entweder um den Wildtyp oder um Mutanten handelt.

3. Verfahren nach Anspruch 2, **dadurch gekennzeichnet, dass** der Wildtyp der Stamm *Yarrowia lipolytica* CBS 6303 ist.

4. Verfahren nach Anspruch 2, **dadurch gekennzeichnet, dass** die Herstellung der Mutanten nach chemischen oder biologischen Methoden erfolgt und bevorzugt durch N-methyl-N'-nitrosoguanidin vorgenommen wird, und dass die Mutante bevorzugt der Stamm *Yarrowia lipolytica* LGX 6481 ist.

5. Verfahren nach eimem der Ansprüche 1 bis 4, **dadurch gekennzeichnet dass** der Induktor gleichzeitig als Kohlenstoffquelle bei der Kultivierung dient und dann in Mengen zwischen 2,5 und 5% v/v eingesetzt wird, bezogen auf das Fermentationsmedium.

6. Verfahren nach eimem der Ansprüche 1 bis 5, **dadurch gekennzeichnet dass** der Induktor in solchem Gehalt eingesetzt wird, dass die Aktivität der Lipase von 1000 U/ml bis zu 2500-3000 U/ml in der Fermenterlösung gesteigert wird.

7. Verwendung von Fettsäureestern kurzkettiger einwertiger Alkohole, ausgewählt aus der Gruppe, die von Methyloleat und Ethyloleat gebildet wird, als Induktoren zur Produktion von extrazellulärer Lipase aus der Hefe *Yarrowia lipolytica.*

8. Verwendung nach Anspruch 7, **dadurch gekennzeichnet, dass** es sich bei der Hefe *Yarrowia lipolytica* entweder um den Wildtyp oder um Mutanten handelt.

## Claims

1. Method for the production of extracellular lipase by means of cultivation of a microorganism, **characterised in that**:
- the microorganism consists of the yeast *yarrowia lipolytica*;
- a fatty acid ester of short-chain, univalent alcohols selected from the group composed of methyl oleate and ethyl oleate is added as inductor so that the content of extracellular lipase in culture supernatants is increased, and
- the so-called inductor is used in quantities between 0.5 and 5% v/v in relation to the fermentation medium.

2. Method according to Claim 1, **characterised in that** the yeast *yarrowia lipolytica* is either the wild type or a mutant.

3. Method according to Claim 2, **characterised in that** the wild type is the strain *yarrowia lipolytica* CBS 6303.

4. Method according to Claim 2, **characterised in that** the manufacture of the mutants is performed according to chemical or biological methods and preferably by means of N-methyl-N'-nitro-N-nitrosoguanidine and that the mutants are preferably the strain *yarrowia lipolytica* LGX 6481.

5. Method according to any of the Claims 1 to 4, **characterised in that** the inductor is used simultaneously as carbon source for the cultivation and is then used in quantities between 2.5 and 5% v/v in relation to the fermentation medium.

6. Method according to any of the Claims 1 to 5, **characterised in that** the inductor is used in such concentration that the activity of the lipase in the fermenter solution is increased from 1000 U/ml up to 2500-3000 U/ml.

7. Use of fatty acid esters of short-chain, univalent alcohols selected from the group composed of methyl oleate and ethyl oleate as inductors for the production of extracellular lipase from the yeast *yarrowia lipolytica.*

8. Use according to Claim 7, **characterised in that** the yeast *yarrowia lipolytica* is either the wild type or a mutant.

## Revendications

1. Procédé de production de lipase exocellulaire par culture d'un microorganisme, **caractérisé en ce que** :
- le microorganisme est la levure *Yarrowia lipolytica,*
- un ester d'acide gras d'alcools monovalentes de chaîne courte choisi dans le groupe composé de l'oléate de méthyle et de l'oléate d'éthyle est ajouté comme inducteur, de façon à élever le taux de lipase exocellulaire dans les cultures et
- ledit inducteur est employé dans des quantités comprises entre 0,5 et 5 % en volume du milieu de fermentation.

2. Procédé selon la revendication 1, **caractérisé en ce que** la levure *Yarrowia lipolytica* est soit du type sauvage, soit de forme mutante.

3. Procédé selon la revendication 2, **caractérisé en ce que** le type sauvage est de la souche *Yarrowia lipolytica* CBS 6303.

4. Procédé selon la revendication 2, **caractérisé en ce que** la fabrication des formes mutantes s'effectue par des procédés chimiques ou biologiques, de préférence à l'aide de N-méthyl-N'-nitrosoguanidine, et la forme mutante est de préférence la souche *Yarrowia lipolytica* LGX 6481.

5. Procédé selon l'une quelconque des revendications 1 à 4, **caractérisé en ce que** l'inducteur sert simultanément de source de carbone pour la culture et est employé dans des quantités comprises entre 2,5 et 5 % en volume du milieu de fermentation.

6. Procédé selon l'une quelconque des revendications 1 à 5, **caractérisé en ce que** l'inducteur est employé dans une proportion telle que l'activité de la lipase augmente de 100 U/ml à 2500-3000 U/ml dans la solution de fermentation.

7. Utilisation d'esters d'acides gras d'alcools monovalents de chaîne courte choisis dans le groupe composé de l'oléate de méthyle et de l'oléate d'éthyle comme inducteurs pour la production de lipase exocellulaire à partir de la levure *Yarrowia lipolytica.*

8. Utilisation selon la revendication 7, **caractérisée en ce que** la levure *Yarrowia lipolytica* est soit du type sauvage, soit de forme mutante.
